# EUROPEAN PATENT SPECIFICATION

(11) **EP 1 806 915 B1**
(45) Date of publication and mention of the grant of the patent: **03.07.2013**
(21) Application number: 06123704.6
(22) Date of filing: 08.11.2006
(51) Int. Cl.: H04N 5/00, H04N 5/63

(54) **Signal processing apparatus and a control method thereof**
Signalverarbeitungsvorrichtung und Kontrollverfahren dafür
Procédé et appareil destinés au traitement de signaux

(30) Priority: 16.11.2005 KR 20050109643
(43) Date of publication of application: 11.07.2007
(73) Proprietor: Samsung Electronics Co., Ltd., Suwon-si, Gyeonggi-do, 443-742 (KR)
(72) Inventor: Lee, Seung-jun, Gyeonggi-do (KR)
(74) Representative: Walaski, Jan Filip

(56) References cited:
- WO-A-00/35190
- US-A- 6 003 131
- US-A1- 2003 009 654
- US-A1- 2003 179 161
- US-B1- 6 560 685

## Description

The present invention relates to a signal processing apparatus and a control method thereof, and more particularly, to a signal processing apparatus that is more convenient for a user to operate and a control method thereof.

A signal processing apparatus, such as a liquid crystal display (LCD) television (TV), receives a video signal and/or an audio signal from a broadcasting station or an external device such as a video cassette recorder (VCR), a digital versatile disk (DVD) player, a personal computer (PC) and so on, processes the received video signal and/or the received audio signal, and then outputs the processed video signal and/or audio signal.

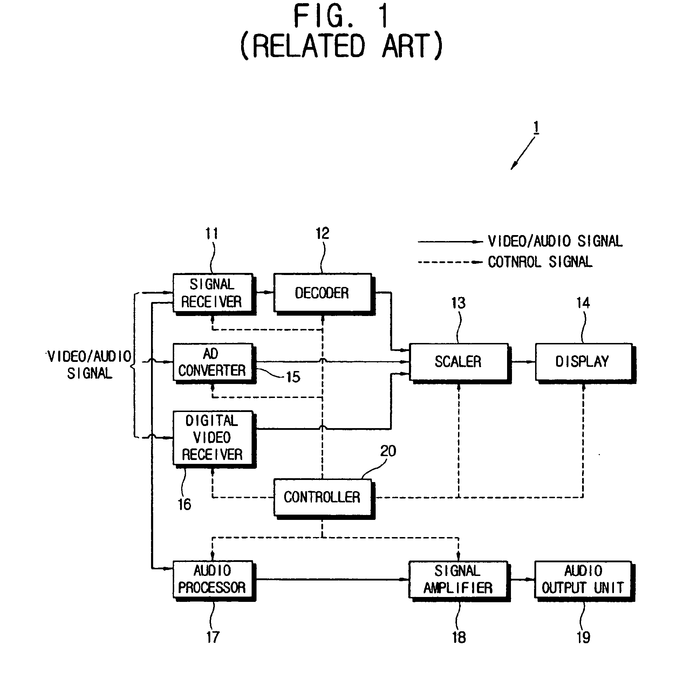

Figure 1 is a schematic block diagram of a related art signal processing apparatus 1. The signal processing apparatus 1 includes a signal receiver 11, a decoder 12, a scaler 13, a display 14, an analog-to-digital (AD) converter 15, a digital video receiver 16, an audio processor 17, a signal amplifier 18, an audio output unit 19, and a controller 20. The signal receiver 11 receives a radio frequency (RF) signal. The decoder 12 receives an analog video signal from the signal receiver 11 and outputs a digital video signal. The AD converter 15 converts the received analog video signal, such as "Component" or "HVRGB", to a digital video signal. The digital video receiver 16 receives a digital video signal such as "DVI/HDMI" and demultiplexes the received digital video signal. The scaler 13 receives the video signal from at least one of the decoder 12, the AD converter 15 and the digital video receiver 16, adjusts resolution of the video according to a characteristic of the display 14 and a user's setting, and outputs the video signal having the adjusted resolution to the display 14. The audio processor 17 receives the audio signal from the signal receiver 11 and decodes the received audio signal, or outputs a received "Audio L/R" signal. The signal amplifier 18 amplifies the audio signal output from the audio processor 17. The audio output unit 19 outputs the audio based on the audio signal received from the signal amplifier 18. The controller 20 controls the overall operation of the components.

As constructed above, the components of the signal processing apparatus 1 can be implemented using integrated circuits (ICs). When the signal processing apparatus 1 is powered on, the components need to be initialized. For example, the components need to be reset by hardware and/or software. Particularly, the signal receiver 11 requires initialization of "phase locked loop (PLL)", "charge pump" and so on. The decoder 12 requires initialization of "input port switching", "clamping", "analog-to-digital converter (ADC) setup" and so on. In many cases, it takes much time for the components to initialize. Especially, when the display 14 is implemented using a LCD panel, after power is supplied to the panel, initialization of backlight (not shown) turn-on, pulse width modulation (PWM) dimming, analog dimming and so on takes a great deal of time in comparison to other components.

However, in the related art signal processing apparatus 1, since the sole controller 20 takes charge of controlling of the other components, there is a disadvantage in that only one command is executed at a time. Thus, upon confirming the powered-on of the signal processing apparatus 1, the controller 20 initializes the components one by one according to a predetermined order. As a result, it takes a considerable time to display the video on a screen after completing the initialization of all the components.

The present invention provides a signal processing apparatus for improving the convenience of a user by shortening an initialization time, and a control method of the signal processing apparatus.

According to an aspect of the present invention, there is provided a signal processing apparatus including a signal receiver which receives a video signal; a video processor which processes the video signal received by the signal receiver; a display which displays a video based on the video signal processed by the video processor; a main controller which determines whether the signal processing apparatus is powered on and initializes the signal receiver and the video processor; and a sub controller which initializes the display while the main controller initializes at least one of the signal receiver and the video processor.

The main controller first initializes one of the signal receiver and the video processor having a longest initialization time. The video processor includes a decoder which decodes the video signal received by the signal receiver. The video processor includes a scaler which scales the video signal received by the signal receiver.

The signal receiver further receives an audio signal, and the apparatus further includes an audio processor which processes the audio signal received by the signal receiver. The signal processing apparatus further includes a signal amplifier which amplifies the audio signal processed by the audio processor. The signal processing apparatus further includes an audio output unit which outputs the audio signal amplified by the signal amplifier. The sub controller initializes at least one of the audio processor and the signal amplifier while the main controller initializes at least one of the signal receiver and the video processor.

The sub controller initializes the display, the audio processor, and the signal amplifier in an order based on initialization times of the display, the audio processor, and the signal amplifier. The sub controller controls the audio processor to process the audio signal which is received by and output from the signal receiver.

According to another aspect of the present invention, there is provided a control method of a signal processing apparatus comprising a signal receiver which receives a video signal, a video processor which processes the video signal received by the signal receiver, and a display which displays the video signal processed by the video processor, the method including determining whether the signal processing apparatus is powered on; initializing the signal receiver and the video processor if it is determined that the signal processing apparatus is powered on; and initializing the display while initializing at least one of the signal receiver and the video processor.

The initializing the signal receiver and the video processor includes first initializing one of the signal receiver and the video processor having a longest initialization time.

The signal receiver further receives an audio signal, the signal processing apparatus further includes an audio processor which processes the audio signal received by the signal receiver, and a signal amplifier which amplifies the audio signal processed by the audio processor, and the control method of the signal processing apparatus further includes initializing at least one of the audio processor and the signal amplifier while initializing at least one of the signal receiver and the video processor.

The initializing the display, the audio processor, and the signal amplifier includes first initializing one of the display, the audio processor, and the signal amplifier having a longest initialization time.

The control method further includes controlling the audio processor to process the audio signal received by and output from the signal receiver.

Embodiments of the invention will now be described, by way of example, with reference to the accompanying drawings, in which:
Figure 1 is a block diagram of a related art signal processing apparatus;
Figure 2 is a schematic block diagram of a signal processing apparatus according to an exemplary embodiment of the present invention; and
Figure 3 is a flow chart illustrating a control method of the signal processing apparatus according to an exemplary embodiment of the present invention.

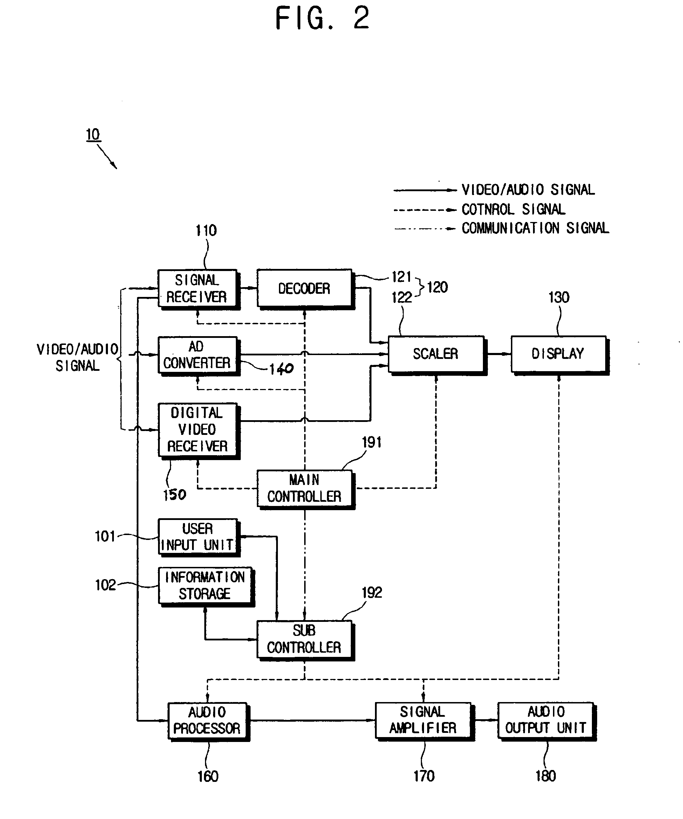

Figure 2 is a schematic block diagram of a signal processing apparatus 10 according to an exemplary embodiment of the present invention. The signal processing apparatus 10 may be a digital TV such as an LCD TV, a plasma display panel (PDP) TV, a digital light processing (DLP) TV and the like. The signal processing apparatus 10 receives a video signal and/or an audio signal from a broadcasting station or an external device such as a VCR, a DVD apparatus, a PC and so on, processes the received video signal and/or the received audio signal, and then outputs the processed video signal and/or audio signal. The signal processing apparatus 10 has two controllers to initialize two or more components in parallel.

Therefore, the convenience of a user can be enhanced by shortening an initialization time.

As shown in Figure 2, the signal processing apparatus 10 includes a signal receiver 110, a video processor 120, a display 130, a main controller 191, and a sub controller 192. The signal receiver 110 receives a radio frequency (RF) video signal. The signal receiver 110 tunes the video signal to a frequency corresponding to a channel selected by a user under control of the main controller 191. The video signal output from the signal receiver 110 may carry a video in an intermediate frequency such as a picture intermediate frequency (PIF).

The video processor 120 receives the video signal from the signal receiver 110 and processes the video signal. The video processor 120 includes a decoder 121 and a scaler 122. The decoder 121 receives an analog video signal from the signal receiver 110 and outputs a digital video signal by performing analog-to-digital (AD) conversion and decoding for the analog video signal.

The scaler 122 receives the video signal from the decoder 121, adjusts resolution of the video according to a characteristic of the display 130 and a user's setting, and outputs the video signal having the adjusted resolution to the display 130.

The display 130 receives the video signal from the scaler 122 and displays the video based on the received video signal. The display 130 may include an LCD panel, a PDP panel, or the like according to the form of display.

The main controller 191 controls the signal receiver 110 and the video processor 120. The main controller 191 initializes the signal receiver 110 and the video processor 120 by checking whether the signal processing apparatus 10 is powered on by the user. The signal processing apparatus 10 may further include a user input unit 101 through which the main controller 191 may receive input information from the user. The user input unit 101 may be implemented by a remote controller.

The main controller 191 initializes the "PLL", "charge pump", "SAW filter", "AGC" of the signal receiver 110, initializes "input port switching", "clamping", "ADC setup", and "output format setup" of the decoder 121, and initializes "input port switching" and "contrast/brightness/colour/tint setup" of the scaler 122.

It may be preferable but not necessary that the main controller 191 first initializes a component having a longer initialization time. For instance, provided that the scaler 122 has the longest initialization time among the signal receiver 110, the decoder 121, and the scaler 122, the main controller 191 may first initialize the scaler 122.

Additionally, the controller 191 can initialize the components based on user setup information. By way of example, the main controller 191 can initialize the components corresponding to information relating to the channel number, the volume, and the screen adjustment state at the time when the user is watching just before the signal processing apparatus is powered down. In this situation, the signal processing apparatus 10 may further include an information storage 102 in which the user setup information is preferably stored.

The sub controller 192 initializes the display 130 in parallel with the main controller 191 initializing the other components, while communicating with the main controller 191. In the case that the signal processing apparatus 10 is an LCD TV, the sub controller 192 initializes "panel power supply", "backlight (not shown) turn-on", "PWM dimming", "analog dimming" of the display 130.

The signal processing apparatus 10 may further include an audio processor 160, a signal amplifier 170, and an audio output unit 180. The audio processor 160 receives the audio signal containing the audio from the signal receiver 110 and performs the audio decoding with respect to the audio signal, or receives and outputs the "audio L/R" signal. The signal amplifier 170 amplifies the audio signal output from the audio processor 160. The audio output unit 180, such as a speaker or the like, outputs the audio based on the audio signal received from the signal amplifier 170.

The sub controller 192 can initialize the audio processor 160 and the signal amplifier 170, as well as the display 130. Particularly, the sub controller 192 initializes "sound port switching", "prescale", "deemphasis", "equalizer", "balance", etc. of the audio processor 160, and "volume", "mute on/off", etc. of the signal amplifier 170.

It is preferable but not necessary that the sub controller 192 initializes the components in order of a longest initialization time to a shortest initialization time, similar to the main controller 191.

The signal processing apparatus 10 further includes an AD converter 140 and a digital video receiver 150 and receives and processes a video signal in various formats. The AD converter 140 converts the received analog video signal, such as "component" and "HVRGB", to the digital video signal. The digital video receiver 150 demultiplexes the received digital video signal such as "DVI/HDMI." The scaler 122 scales the video signals output from the AD converter 140 and the digital video receiver 150.

After the completion of the initialization, the main controller 191 and the sub controller 192 control the components to receive the video signal and/or the audio signal, and output the video and/or audio with respect to the received signal(s). In more detail, the main controller 191 and the sub controller 192 set up the respective corresponding components. In this case, the main controller 191 and the sub controller 192 perform the component setup based on their respective input signals.

For example, the main controller 191 sets up "PLL", "charge pump", "SAW filter", and "AGC" of the signal receiver 110, "input signal standard recognition", "filter setup", and "mixer setup" of the decoder 121, and "capture/display setup" and "scaling coefficient setup" of the scaler 122, and the sub controller 192 sets up "input signal standard recognition" and "filter setup" of the audio processor 160.

At this time, the sub controller 192 checks whether the setup of the signal receiver 110 is completed using the information received from the main controller 191, and then sets up the audio processor 160 based on the audio signal output from the signal receiver 110. The audio signal output from the signal receiver 110 may contain the audio in an intermediate frequency such as a sound intermediate frequency (SIF).

It is noted that the main controller 191 and the sub controller 192 can be implemented by computer programs executed by a microprocessor such as a central processing unit (CPU).

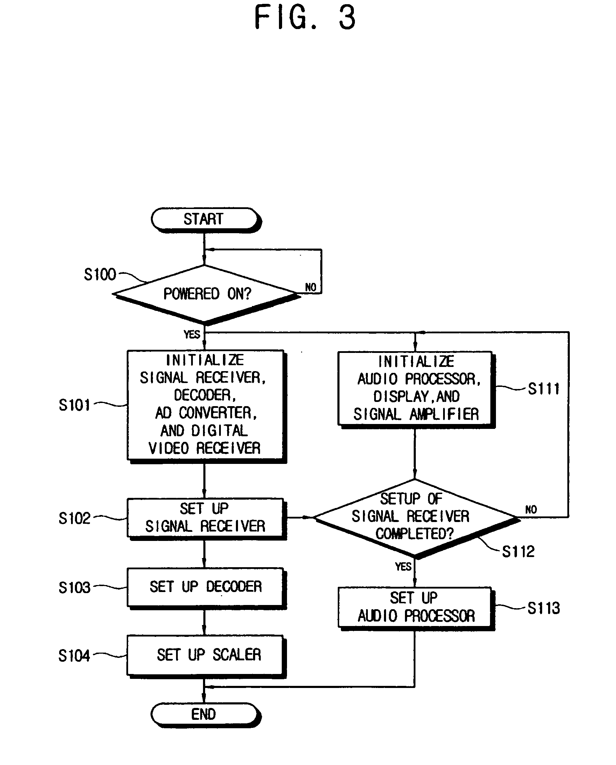

Figure 3 is a flow chart illustrating a control method of the signal processing apparatus 10 according to an exemplary embodiment of the present invention. First, it is determined whether the signal processing apparatus 10 is powered on by the user at operation S100. If it is determined that the signal processing apparatus 10 is powered on, as a unit of the main control, the signal receiver 110, the decoder 121, the AD converter 140, and the digital video receiver 150 of the signal processing apparatus 150 are initialized at operation S101. As a unit of the sub-control in temporal-parallel with the main control, the audio processor 160, the display 130, and the signal amplifier 170 of the signal processing apparatus 10 are initialized at operation S111.

Upon completing the initialization of the signal receiver 110, the decoder 121, the AD converter 140, and the digital video receiver 150, as a unit of the main control, the signal receiver 110 is set up based on the input video signal at operation S102. Upon completing the setup of the signal receiver 110, the decoder 121 is set up based on the video signal output from the signal receiver 110 at operation S103. After the setup of the decoder 121, the scaler 122 is set up based on the video signal output from the decoder 121 at operation S104.

Finally, as a unit of the sub-controller, it is determined whether the setup of the signal receiver 110 is completed at operation S112. If it is determined that the set up of the signal receiver 110 is completed, the audio processor 160 is set up based on the audio signal output from the signal receiver 110 at operation S113.

Accordingly, the present invention provides a signal processing apparatus for improving the convenience of a user by shortening an initialization time, and a control method thereof.

Although a few exemplary embodiments of the present invention have been shown and described, it will be appreciated by those skilled in the art that changes may be made in these embodiments without departing from the principles of the invention, the scope of which is defined in the appended claims.

## Claims

1. A signal processing apparatus comprising:
a signal receiver (110) for receiving a video signal;
a video processor (120) for processing the video signal received by the signal receiver;
a display (130) for displaying video corresponding to the video signal;
a first controller (191) operable to initialize the signal receiver and the video processor in response to the signal processing apparatus being powered on; and
a second controller (192) arranged to communicate with and to operate in parallel with the first controller, and arranged to initialize the display while the first controller initializes at least one of the signal receiver and the video processor;
wherein the first controller is arranged to communicate information relating to the progress of initialisation of the signal receiver and the video processor to the second controller; and
the second controller is arranged to communicate user setup information to the first controller.

2. A signal processing apparatus according to claim 1, wherein the first controller is arranged to first initialize the one of the signal receiver and the video processor having a longer initialization time.

3. A signal processing apparatus according to claim 1 or 2, wherein the video processor (120) comprises a decoder (121) arranged to decode the video signal received by the signal receiver.

4. A signal processing apparatus according to claim 1, 2 or 3, wherein the video processor (120) comprises a scaler (122) which scales the video signal received by the signal receiver.

5. A signal processing apparatus according to any preceding claim, wherein the signal receiver (110) is further arranged to receive an audio signal, and
wherein the apparatus further comprises an audio processor (160) for processing the audio signal received by the signal receiver.

6. A signal processing apparatus according to claim 5, further comprising a signal amplifier (170) arranged to amplify the audio signal processed by the audio processor (160).

7. A signal processing apparatus according to claim 6, further comprising an audio output unit (180) arranged to outputs the audio signal amplified by the signal amplifier (170).

8. A signal processing apparatus according to claim 6 or 7, wherein the second controller (192) is arranged to initialize at least one of the audio processor (160) and the signal amplifier (170) while the first controller (191) initializes at least one of the signal receiver (110) and the video processor (120).

9. A signal processing apparatus according to claim 8, wherein the second controller (192) is arranged to initialize the display (130), the audio processor (160), and the signal amplifier (170) in an order based on initialization times of the display, the audio processor, and the signal amplifier.

10. A signal processing apparatus according to any one of claims 5 to 9, wherein the second controller (192) is arranged to control the audio processor to process the audio signal which is received by and output from the signal receiver (110).

11. A signal processing apparatus according to any one of claims 5 to 10 in which the first controller (191) is arranged to configure the signal receiver (110) based on the received video signal, after initialization of the signal receiver.

12. A signal processing apparatus according to claim 11 in which the second controller (192) is arranged to configure the audio processor (160) based on the received audio signal in response to determining that the signal receiver has been configured by the first controller (191).

13. A control method of a signal processing apparatus comprising a signal receiver (110) which receives a video signal, a video processor (120) which processes the video signal received by the signal receiver, and a display (130) which displays the video signal processed by the video processor, the method comprising:
determining whether the signal processing apparatus is powered on;
a first controller (191) initializing the signal receiver and the video processor if it is determined that the signal processing apparatus is powered on; and
a second controller (192), communicating with and operating in parallel with the first controller, initializing the display while the first controller is initializing at least one of the signal receiver and the video processor,
wherein the first controller communicates information relating to the progress of initialisation of the signal receiver and the video processor to the second controller; and
the second controller communicates user setup information to the first controller.

14. A control method according to claim 13, wherein the initializing the signal receiver (110) and the video processor (120) comprises initializing first the one of the signal receiver and the video processor having a longer initialization time.

15. A control method according to claim 12 or 13, wherein the signal receiver further receives an audio signal, wherein the signal processing apparatus further comprises an audio processor (160) which processes the audio signal received by the signal receiver (110), and a signal amplifier (170) which amplifies the audio signal processed by the audio processor, and wherein the control method further comprises initializing at least one of the audio processor and the signal amplifier while initializing the signal receiver and the video processor.

16. A control method according to claim 15, wherein the initializing the display (130), the audio processor (160), and the signal amplifier (170) comprises initializing first the one of the display, the audio processor, and the signal amplifier having a longest initialization time.

17. A control method according to claim 15 or 16, further comprising controlling the audio processor (160) to process the audio signal received in and output from the signal receiver (110).

18. A control method according to any one of claims 15 to 17 in which the first controller (191) configures the signal receiver (110) based on the received video signal, after initialization of the signal receiver.

19. A control method according to claim 18 in which the second controller (192) configures the audio processor (160) based on the received audio signal in response to determining that the signal receiver has been configured by the first controller (191).

## Patentansprüche

1. Signalverarbeitungsvorrichtung, die Folgendes umfasst:
einen Signalempfänger (110) zum Empfangen eines Videosignals;
einen Videoprozessor (120) zum Verarbeiten des vom Signalempfänger empfangenen Videosignals;
ein Display (130) zum Anzeigen von dem Videosignal entsprechendem Video;
eine erste Steuerung (191) zum Initialisieren des Signalempfängers und des Videoprozessors als Reaktion auf das Einschalten der Signalverarbeitungsvorrichtung; und
eine zweite Steuerung (192) zum Kommunizieren mit und zum Arbeiten parallel zu der ersten Steuerung und zum Initialisieren des Displays, während die erste Steuerung den Signalempfänger und/oder den Videoprozessor initialisiert;
wobei die erste Steuerung zum Übermitteln von Informationen über den Fortschritt des Initialisierens des Signalempfängers und des Videoprozessors zur zweiten Steuerung ausgelegt ist; und
die zweite Steuerung zum Übermitteln von Benutzer-Setup-Informationen zur ersten Steuerung ausgelegt ist.

2. Signalverarbeitungsvorrichtung nach Anspruch 1, wobei die erste Steuerung so ausgelegt ist, dass sie zunächst denjenigen aus Signalempfänger und Videoprozessor mit der längeren Initialisierungszeit initialisiert.

3. Signalverarbeitungsvorrichtung nach Anspruch 1 oder 2, wobei der Videoprozessor (120) einen Decoder (121) zum Decodieren des vom Signalempfänger empfangenen Videosignals umfasst.

4. Signalverarbeitungsvorrichtung nach Anspruch 1, 2 oder 3, wobei der Videoprozessor (120) einen Skalierer (122) umfasst, der das vom Signalempfänger empfangene Videosignal skaliert.

5. Signalverarbeitungsvorrichtung nach einem der vorherigen Ansprüche, wobei der Signalempfänger (110) ferner zum Empfangen eines Audiosignals ausgelegt ist, und
wobei die Vorrichtung ferner einen Audioprozessor (160) zum Verarbeiten des vom Signalempfänger empfangenen Audiosignals umfasst.

6. Signalverarbeitungsvorrichtung nach Anspruch 5, die ferner einen Signalverstärker (170) zum Verstärken des vom Audioprozessor (160) verarbeiteten Audiosignals umfasst.

7. Signalverarbeitungsvorrichtung nach Anspruch 6, die ferner eine Audioausgabeeinheit (180) zum Ausgeben des vom Signalverstärker (170) verstärkten Audiosignals umfasst.

8. Signalverarbeitungsvorrichtung nach Anspruch 6 oder 7, wobei die zweite Steuerung (192) zum Initialisieren des Audioprozessors (160) und/oder des Signalempfängers (170) ausgelegt ist, während die erste Steuerung (191) den Signalempfänger (110) und/oder den Videoprozessor (120) initialisiert.

9. Signalverarbeitungsvorrichtung nach Anspruch 8, wobei die zweite Steuerung (192) zum Initialisieren des Displays (130), des Audioprozessors (160) und des Signalverstärkers (170) in einer Reihenfolge ausgelegt ist, die auf Initialisierungszeiten des Displays, des Audioprozessors und des Signalverstärkers basiert.

10. Signalverarbeitungsvorrichtung nach einem der Ansprüche 5 bis 9, wobei die zweite Steuerung (192) zum Steuern des Audioprozessors zum Verarbeiten des vom Signalempfänger (110) empfangenen und davon ausgegebenen Audiosignals ausgelegt ist.

11. Signalverarbeitungsvorrichtung nach einem der Ansprüche 5 bis 10, wobei die erste Steuerung (191) zum Konfigurieren des Signalempfängers (110) auf der Basis des empfangenen Videosignals nach dem Initialisieren des Signalempfängers ausgelegt ist.

12. Signalverarbeitungsvorrichtung nach Anspruch 11, wobei die zweite Steuerung (192) zum Konfigurieren des Audioprozessors (160) auf der Basis des empfangenen Audiosignals als Reaktion auf die Ermittlung ausgelegt ist, dass der Signalempfänger von der ersten Steuerung (191) konfiguriert wurde.

13. Verfahren zum Steuern einer Signalverarbeitungsvorrichtung, die einen Signalempfänger (110) zum Empfangen eines Videosignals, einen Videoprozessor (120) zum Verarbeiten des vom Signalempfänger empfangenen Videosignals und ein Display (130) zum Anzeigen des vom Videoprozessor verarbeiteten Videosignals umfasst, wobei das Verfahren Folgendes beinhaltet:
Ermitteln, ob die Signalverarbeitungsvorrichtung eingeschaltet ist;
Initialisieren, durch eine erste Steuerung (191), des Signalempfängers und des Videoprozessors, wenn festgestellt wird, dass die Signalverarbeitungsvorrichtung eingeschaltet ist; und
Kommunizieren, durch eine zweite Steuerung (192), mit und Arbeiten parallel zu der ersten Steuerung, Initialisieren des Displays, während die erste Steuerung den Signalempfänger und/oder den Videoprozessor initialisiert,
wobei die erste Steuerung Informationen über den Fortschritt des Initialisierens des Signalempfängers und des Videoprozessors zur zweiten Steuerung übermittelt; und
die zweite Steuerung Benutzer-Setup-Informationen zur ersten Steuerung übermittelt.

14. Steuerverfahren nach Anspruch 13, wobei das Initialisieren des Signalempfängers (110) und des Videoprozessors (120) beinhaltet, dass zunächst derjenige aus Signalempfänger und Videoprozessor mit der längeren Initialisierungszeit initialisiert wird.

15. Steuerverfahren nach Anspruch 12 oder 13, wobei der Signalempfänger ferner ein Audiosignal empfängt, wobei die Signalverarbeitungsvorrichtung ferner einen Audioprozessor (160), der das vom Signalempfänger (110) empfangene Audiosignal verarbeitet, und einen Signalverstärker (170) umfasst, der das vom Audioprozessor verarbeitete Audiosignal verstärkt, und wobei das Steuerverfahren ferner das Initialisieren des Audioprozessors und/oder des Signalverstärkers beinhaltet, während der Signalempfänger und der Videoprozessor initialisiert werden.

16. Steuerverfahren nach Anspruch 15, wobei das Initialisieren des Displays (130), des Audioprozessors (160) und des Signalverstärkers (170) das Initialiseren zunächst desjenigen aus Display, Audioprozessor und Signalverstärker mit der längsten Initialisierungszeit beinhaltet.

17. Steuerverfahren nach Anspruch 15 oder 16, das ferner das Steuern des Audioprozessors (160) zum Verarbeiten des im Signalempfänger (110) empfangenen und davon ausgegebenen Audiosignals beinhaltet.

18. Steuerverfahren nach einem der Ansprüche 15 bis 17, wobei die erste Steuerung (191) den Signalempfänger (110) auf der Basis des empfangenen Videosignals nach dem Initialisieren des Signalempfängers konfiguriert.

19. Steuerverfahren nach Anspruch 18, wobei die zweite Steuerung (192) den Audioprozessor (160) auf der Basis des empfangenen Audiosignals als Reaktion auf die Feststellung konfiguriert, dass der Signalempfänger von der ersten Steuerung (191) konfiguriert wurde.

## Revendications

1. Appareil de traitement de signal comprenant :
un récepteur de signal (110) pour recevoir un signal vidéo ;
un processeur vidéo (120) pour traiter le signal vidéo reçu par le récepteur de signal :
un afficheur (130) pour afficher la vidéo correspondant au signal vidéo ;
une première unité de commande (191) exploitable pour initialiser le récepteur de signal et le processeur vidéo en réponse à la mise sous tension de l'appareil de traitement de signal ; et
une seconde unité de commande (192) agencée pour communiquer et fonctionner en parallèle avec la première unité de commande, et agencée pour initialiser l'afficheur pendant que la première unité de commande initialise au moins l'un du récepteur de signal et du processeur vidéo ;
dans lequel la première unité de commande est agencée pour communiquer à la seconde unité de commande des informations relatives à la progression de l'initialisation du récepteur de signal et du processeur vidéo ; et
la seconde unité de commande est agencée pour communiquer à la première unité de commande des informations de configuration d'utilisateur.

2. Appareil de traitement de signal selon la revendication 1, dans lequel la première unité de commande est agencée pour d'abord initialiser celui du récepteur de signal et du processeur vidéo qui a un plus long temps d'initialisation.

3. Appareil de traitement de signal selon la revendication 1 ou 2, dans lequel le processeur vidéo (120) comprend un décodeur (121) agencé pour décoder le signal vidéo reçu par le récepteur de signal.

4. Appareil de traitement de signal selon la revendication 1, 2 ou 3, dans lequel le processeur vidéo (120) comprend un échelonneur (122) qui met à l'échelle le signal vidéo reçu par le récepteur de signal.

5. Appareil de traitement de signal selon l'une quelconque des revendications précédentes, dans lequel le récepteur de signal (110) est agencé en outre pour recevoir un signal audio, et
dans lequel l'appareil comprend en outre un processeur audio (160) pour traiter le signal audio reçu par le récepteur de signal.

6. Appareil de traitement de signal selon la revendication 5, comprenant en outre un amplificateur de signal (170) agencé pour amplifier le signal audio traité par le processeur audio (160).

7. Appareil de traitement de signal selon la revendication 6, comprenant en outre une unité de sortie audio (180) agencée pour produire en sortie le signal audio amplifié par l'amplificateur de signal (170) .

8. Appareil de traitement de signal selon la revendication 6 ou 7, dans lequel la seconde unité de commande (192) est agencée pour initialiser au moins l'un du processeur audio (160) et de l'amplificateur de signal (170) pendant que la première unité de commande (191) initialise au moins l'un du récepteur de signal (110) et du processeur vidéo (120).

9. Appareil de traitement de signal selon la revendication 8, dans lequel la seconde unité de commande (192) est agencée pour initialiser l'afficheur (130), le processeur audio (160), et l'amplificateur de signal (170) dans un ordre basé sur des temps d'initalisation de l'afficheur, le processeur audio et l'amplificateur de signal.

10. Appareil de traitement de signal selon l'une quelconque des revendications 5 à 9, dans lequel la seconde unité de commande (192) est agencée pour commander le processeur audio afin de traiter le signal audio qui est reçu par le récepteur de signal et produit en sortie depuis celui-ci (110).

11. Appareil de traitement de signal selon l'une quelconque des revendications 5 à 10, dans lequel la première unité de commande (191) est agencée pour configurer le récepteur de signal (110) en fonction du signal vidéo reçu, après l'initialisation du récepteur de signal.

12. Appareil de traitement de signal selon la revendication 11, dans lequel la seconde unité de commande (192) est agencée pour configurer le processeur audio (160) en fonction du signal audio reçu en réponse à la détermination que le récepteur de signal a été configuré par la première unité de commande (191).

13. Procédé de commande d'un appareil de traitement de signal comprenant un récepteur de signal (110) qui reçoit un signal vidéo, un processeur vidéo (120) qui traite le signal vidéo reçu par le récepteur de signal, et un afficheur (130) qui affiche le signal vidéo traité par le processeur vidéo, le procédé comprenant :
la détermination que l'appareil de traitement de signal est sous tension ou non ;
une première unité de commande (191) qui initialise le récepteur de signal et le processeur vidéo s'il est déterminé que l'appareil de traitement de signal est sous tension ; et
une seconde unité de commande (192) qui communique et fonctionne en parallèle avec la première unité de commande, initialise l'afficheur pendant que la première unité de commande initialise au moins l'un du récepteur de signal et du processeur vidéo ;
dans lequel la première unité de commande communique à la seconde unité de commande des informations relatives à la progression de l'initialisation du récepteur de signal et du processeur vidéo ; et
la seconde unité de commande communique à la première unité de commande des informations de configuration d'utilisateur.

14. Procédé de commande selon la revendication 13, dans lequel l'initialisation du récepteur de signal (110) et du processeur vidéo (120) comprend l'initialisation en premier de celui du récepteur de signal et du processeur vidéo qui a un plus long temps d'initialisation.

15. Procédé de commande selon la revendication 12 ou 13, dans lequel le récepteur de signal reçoit en outre un signal audio, dans lequel l'appareil de traitement de signal comprend en outre un processeur audio (160) qui traite le signal audio reçu par le récepteur de signal (110), et un amplificateur de signal (170) qui amplifie le signal audio traité par le processeur audio, et le procédé de commande comprenant en outre l'initialisation d'au moins l'un du processeur audio et de l'amplificateur de signal durant l'initialisation du récepteur de signal et du processeur vidéo.

16. Procédé de commande selon la revendication 15, dans lequel l'initialisation de l'afficheur (130), du processeur audio (160) et de l'amplificateur de signal (170) comprend l'initialisation en premier de celui de l'afficheur, du processeur audio et de l'amplificateur de signal qui a le plus long temps d'initialisation.

17. Procédé de commande selon la revendication 15 ou 16, comprenant en outre la commande du processeur audio (160) pour qu'il traite le signal audio reçu dans le récepteur de signal (110) et produit en sortie depuis celui-ci.

18. Procédé de commande selon l'une quelconque des revendications 15 à 17, dans lequel la première unité de commande (191) configure le récepteur de signal (110) en se basant sur le signal vidéo reçu, après l'initialisation du récepteur de signal.

19. Procédé de commande selon la revendication 18, dans lequel la deuxième unité de commande (192) configure le processeur audio (160) en se basant sur le signal audio reçu en réponse à la détermination que le récepteur de signal a été configuré par la première unité de commande (191).
